# EUROPÄISCHE PATENTSCHRIFT

(11) **EP 1 430 829 B1**
(45) Veröffentlichungstag und Bekanntmachung des Hinweises auf die Patenterteilung: **09.04.2008**
(21) Anmeldenummer: 02406102.0
(22) Anmeldetag: 16.12.2002
(51) Int. Cl.: A61B 3/10, A61B 3/107, G01B 11/24, G01B 11/255

(54) **Opthalmologische Vorrichtung und opthalmologisches Messverfahren**
Ophthalmic device and measuring method
Dispositif et méthode de mesure ophtalmologique

(43) Veröffentlichungstag der Anmeldung: 23.06.2004
(73) Patentinhaber: SIS AG Surgical Instrument Systems, 2555 Brügg b. Biel (CH)
(72) Erfinder: Rathjen, Dr.Ing. Christian, 28197 Bremen (DE)
(74) Vertreter: Vogel, Dany

(56) Entgegenhaltungen:
- EP-A- 0 933 060
- US-A- 4 711 541
- US-A- 5 202 708
- US-A- 5 341 180

## Beschreibung

### Technisches Gebiet

Die vorliegende Erfindung betrifft eine opthalmologische Vorrichtung und ein opthalmologisches Messverfahren. Die Erfindung betrifft insbesondere eine opthalmologische Vorrichtung und ein opthalmologisches Messverfahren, in welchen mittels eines Lichtprojektors ein Strahlenbündel durch einen Querschnittsteil eines Auges projiziert wird, insbesondere durch einen Querschnittsteil der Hornhaut, in welchen mittels erster Bilderfassungsmittel, die in Scheimpfluganordnung zum Strahlenbündel angeordnet sind, ein Querschnittabbild von mindestens einem Teilgebiet des durch den Lichtprojektor beleuchteten Querschnittsteils aus einer ersten Position ausserhalb des Strahlenbündels erfasst und gespeichert wird, und in welchen mittels zweiter Bilderfassungsmittel ein Ansichtabbild des Auges erfasst und dem erfassten Querschnittabbild zugeordnet gespeichert wird.

### Stand der Technik

Im Stand der Technik sind opthalmologische Vorrichtungen und opthalmologische Messverfahren bekannt, in welchen mittels eines Lichtprojektors ein Strahlenbündel durch einen Querschnittsteil eines Auges, insbesondere durch einen Querschnittsteil der Hornhaut, projiziert wird. Typischerweise wird das Strahlenbündel in Form eines Lichtspalts projiziert. In der Patentschrift US 5404884 werden ein Verfahren und eine Vorrichtung für die Untersuchung von Hornhautgewebe eines Patienten beschrieben. Gemäss US 5404884 wird ein im Wesentlichen ebener Laserstrahl mit einem spaltförmigen Profil durch einen Querschnittsteil der Hornhaut projiziert. Durch die Erfassung von mindestens einem Teil des in der Hornhaut gestreuten Lichts, das heisst von mindestens einem Teil des Lichtspalts, wird gemäss US 5404884 ein Querschnittabbild der Hornhaut gewonnen. Aus mehreren solchen Querschnittabbildern der Hornhaut können gemäss US 5404884 Hornhauttrübungen, Hornhautdicke und Hornhauttopografie umfassend für die gesamte Hornhaut bestimmt werden. Da sich die Augen relativ zu der Untersuchungsvorrichtung bewegen können, kann die Untersuchung des gesamten Auges gemäss US 5404884 zu Ungenauigkeiten führen, weil diese Relativbewegungen nicht erfasst und berücksichtigt werden. Im Fachartikel B. R. Masters et al. ,"Transformation of a Set of Slices Rotated on a Common Axis to a Set of Z-Slices: Application to Three-Dimensional Visualization of the In Vivo Human Lens", Computerized Medical Imaging and Graphics, Vol. 21, No. 3, Seiten 145 bis 151, 1997, wird überdies explizit darauf hingewiesen, dass sich bei umfassender Untersuchung des Auges, die auf der Zusammenfügung von mehreren Querschnittabbildern basiert, bedingt durch die Schwierigkeit der gegenseitigen Ausrichtung der einzelnen Querschnittabbilder Messartefakte ergeben können.

In der Patentschrift US 4711541 wird eine opthalmologische Vorrichtung beschrieben, die über eine Spaltlampe zur Projektion eines Lichtspalts auf die Linse eines Auges verfügt. Die Vorrichtung gemäss US 4711541 umfasst zudem eine Fotokamera, die bezüglich der Ebene des Lichtspalts gemäss Scheimpflugbedingungen angeordnet ist, um den gesamten Querschnittsteil der Augenlinse, der durch den Lichtspalt beleuchtet wird, scharf abzubilden. Die Vorrichtung gemäss US 4711541 verfügt über ein Stereomikroskop, um dem Benutzer eine Aufsicht auf das Auge zu ermöglichen. Mittels optischer Elemente der Vorrichtung kann die Aufsicht zur Abbildung der Fotokamera zugeführt werden. Dabei wird jedoch mittels Polarisationsfiltern sichergestellt, dass nicht der auf der Oberfläche der Augenlinse reflektierte Lichtspalt, sondern nur die in der Aufsicht sichtbare Hintergrundbeleuchtung des Auges, die von der Reflektion des Lichtspalts am Augenhintergrund und der Streuung durch die Augenlinse herrührt, der Fotokamera zur Abbildung zugeführt wird. Mittels eines beweglichen Spiegels können der Lichtschnitt in der Augenlinse und die Aufsicht des Auges mit der Hintergrundbeleuchtung nebeneinander auf dieselbe Fotografie abgebildet werden. Da die Vorrichtung gemäss US 4711541 nur eine Untersuchung mit Einzelbildern ermöglicht, kann keine zusammenhängende Untersuchung des gesamten Auges durchgeführt werden.

In der Patentschrift US 5341180 wird eine opthalmologische Bildaufnahmevorrichtung beschrieben, welche mittels einer Spaltlampe ein Lichtspalt auf ein Auge projiziert. Die Bildaufnahmevorrichtung umfasst eine CCD-Kamera (Charged Coupled Device) welche zur Ebene des Lichtspalts entsprechend Scheimpflugbedingungen angeordnet ist, um den gesamten Querschnittsteil des Auges, der durch den Lichtspalt beleuchtet wird, scharf abzubilden. Die Vorrichtung gemäss US 5341180 umfasst eine zweite CCD-Kamera, die dem Benutzer eine Aufsicht auf das zu untersuchende Auge gewährt und zur Ausrichtung der Vorrichtung respektive des Auges mit Hilfe von auf das Auge projizierten Lichtmarken dient. Die Vorrichtung gemäss US 5341180 verfügt über Polarisationsfilter, um zu verhindern, dass der Lichtspalt in der Aufsicht der zweiten CCD-Kamera sichtbar ist. Um präzise Ausrichtungen zu ermöglichen, muss der zu untersuchende Patient bei jeder Aufnahme seine Augen auf Fixiermarken fokussieren, was bei einer Untersuchung des gesamten Auges vom Patienten als mühsam empfunden werden kann und auch zeitaufwendig ist.

In EP 0933060 wird eine opthalmologische Vorrichtung beschrieben, die eine Spaltlampe zur Projektion eines Lichtspalts auf ein Auge, eine CCD Kamera zur Erfassung eines Querschnittabbilds sowie eine CCD Kamera zur Erfassung eines Ansichtabbilds des Auges umfasst. In der Vorrichtung nach EP 0933060 werden das Ansichtabbild und das Querschnittabbild in verschiedenen Framespeichern gespeichert, die mit einem Bildwechsler verbunden sind. Nach EP 0933060 werden entweder das Ansichtabbild oder das Querschnittabbild auf einer Anzeige dargestellt. Die Ansichtabbilder werden nach EP 0933060 zur Ausrichtung der Vorrichtung aufgenommen und die Vorrichtung wird nach erfolgter Ausrichtung mittels eines dafür vorgesehenen Schalter in einen speziellen Modus geschaltet, in welchem das Querschnittabbild aufgenommen wird.

Eine weitere opthalmologische Vorrichtung, die eingerichtet ist ein Querschnittabbild eines auf ein Auge projizierten Lichtspalts aufzunehmen, wird in der Patentschrift US 5208708 beschrieben. Auch nach US 5208708 wird zur Ausrichtung der Vorrichtung ein Ansichtabbild verwendet und die Aufnahme des Querschnittabbilds erfolgt nach Beendigung der Ausrichtung.

### Darstellung der Erfindung

Es ist eine Aufgabe der vorliegenden Erfindung, eine neue opthalmologische Vorrichtung und ein neues opthalmologisches Messverfahren vorzuschlagen, welche nicht die Nachteile des Stand der Technik aufweisen und die insbesondere eine zusammenhängende Untersuchung des gesamten Auges ermöglichen, insbesondere die Bestimmung von Topografie und Messwerten von Strukturen der Vorderkammer des Auges, beispielsweise die Hornhauttopografie und -dicke, wobei Relativbewegungen des Auges zur Vorrichtung berücksichtigt werden.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die opthalmologische Vorrichtung umfasst einen ersten Lichtprojektor zur Projektion eines Strahlenbündels durch einen Querschnittsteil eines Auges, insbesondere durch einen Querschnittsteil der Hornhaut des Auges, erste Bilderfassungsmittel zur Erfassung und Speicherung eines Querschnittabbilds von mindestens einem Teilgebiet des durch den ersten Lichtprojektor beleuchteten Querschnittsteils, aus einer ersten Position ausserhalb des Strahlenbündels, welche in Scheimpfluganordnung zum Strahlenbündel angeordnet sind, und zweite Bilderfassungsmittel zur Erfassung eines Ansichtabbilds des Auges und zur Speicherung des erfassten Ansichtabbilds zugeordnet zum erfassten Querschnittabbild.

Die oben genannten Ziele werden durch die Erfindung insbesondere dadurch erreicht, dass die zweiten Bilderfassungsmittel dieser opthalmologischen Vorrichtung eingerichtet sind zur Erfassung und Speicherung des Ansichtabbilds derart, dass das Ansichtabbild ein Abbild des durch den ersten Lichtprojektor beleuchteten Querschnittsteils umfasst, und dass diese opthalmologische Vorrichtung Verarbeitungsmittel umfasst zur Positionierung des gespeicherten Querschnittabbilds relativ zum Auge auf der Basis des zugeordnet gespeicherten Ansichtabbilds. Die Erfassung und Speicherung des Querschnittabbilds und des dazu gehörenden Ansichtabbilds mit dem beleuchteten Querschnittsteil ermöglicht die Bestimmung der Position des Querschnittabbilds respektive des darin erfassten beleuchteten Querschnittsteils relativ zum Auge auf der Basis des zugeordneten Ansichtabbilds, was wiederum eine zusammenhängende Untersuchung des gesamten Auges mit mehreren Querschnittabbildern ermöglicht, wobei Relativbewegungen des Auges auf Grund der bestimmten Positionen der betreffenden Querschnittabbilder berücksichtigt werden können. Als Bezugspunkte für die Positionsbestimmung können natürliche Merkmale des Auges wie Limbus, Iris oder Pupille, verwendet werden, die im Ansichtabbild abgebildet sind. Da zu jedem Querschnittabbild automatisch auch die relative Position bestimmt wird, kann das gesamte Auge zusammenhängend untersucht werden, indem mehrere Querschnittabbilder entsprechend ihrer zugeordneten Position zu einem dreidimensionalen Abbild des Auges zusammengefügt werden. Es wird eine zusammenhängende Untersuchung des gesamten Auges ermöglicht, bei der Relativbewegungen des Auges zur Vorrichtung berücksichtigt werden, ohne dass der zu untersuchende Patient bei jeder Aufnahme seine Augen auf Fixiermarken fokussieren muss, um Messfehler zu vermeiden. Aus mehreren Querschnittabbildern, die entsprechend ihrer bestimmten Position zusammengefügt werden, können beispielsweise Hornhautdicke, Hornhauttopografie und/oder Hornhauttrübungen umfassend für die gesamte Hornhaut des Auges bestimmt werden. Die Verarbeitungsmittel sind vorzugsweise eingerichtet zur Positionierung mehrerer gespeicherter Querschnittabbilder relativ zueinander auf der Basis der ihnen jeweils zugeordneten gespeicherten Ansichtabbilder.

In einer Ausführungsvariante sind die Verarbeitungsmittel eingerichtet zum Bestimmen der Dicke des durch den ersten Lichtprojektor beleuchteten Querschnittsteils des Auges auf der Basis des gespeicherten Ansichtabbilds. Da das vom ersten Lichtprojektor projizierte Strahlenbündel eine endliche Dicke aufweist und divergent sein kann, erscheint die Dicke des beleuchteten Querschnittsteils im ausserhalb des Strahlenbündels erfassten Querschnittabbild abhängig von der Dicke des Strahlenbündels grösser oder kleiner. Die Bestimmung der Dicke des durch den ersten Lichtprojektor beleuchteten Querschnittsteils des Auges hat den Vorteil, dass der Einfluss der endlichen Dicke des Strahlenbündels bei der Dickenmessung von beleuchteten Querschnittsteilen des Auges, beispielsweise bei der Hornhautdickenmessung, berücksichtigt und die Dickenmessung entsprechend korrigiert werden kann, was zu höherer Messgenauigkeit führt. Für die Bestimmung der Dicke des durch den ersten Lichtprojektor beleuchteten Querschnittsteils respektive des Strahlenbündels erweist sich ein Aufsichtabbild, also ein Ansichtabbild, bei dem die zweiten Bilderfassungsmittel so angeordnet werden, dass ihre optische Achse im Wesentlichen parallel zu der optischen Achse oder der Sehachse des Auges verläuft oder mit der optischen Achse oder der Sehachse des Auges zusammenfällt, als besonders vorteilhaft, weil besonders genau und einfach, wenn die optische Achse der zweiten Bilderfassungsmittel mit dem durch den Querschnittsteil verlaufenden Strahlenbündel zusammenfällt.

Die bevorzugte Anordnung der zweiten Bilderfassungsmittel und des ersten Lichtprojektors, in welcher die optische Achse der zweiten Bilderfassungsmittel mit dem durch den Querschnittsteil verlaufenden Strahlenbündel zusammenfällt, ermöglicht in vorteilhafter Weise auch eine besonders einfache und genaue Positionsbestimmung des abgebildeten beleuchteten Querschnittsteils, insbesondere wenn das Auge durch die zweiten Bilderfassungsmittel als Aufsichtabbild erfasst wird.

In einer bevorzugten Ausführungsvariante sind die ersten und die zweiten Bilderfassungsmittel so angeordnet, dass ihre optischen Achsen in einer gemeinsamen Ebene liegen. Durch diese Anordnung können die durch ersten Bilderfassungsmittel erfassten Querschnittabbilder und die durch zweiten Bilderfassungsmittel erfassten zugeordneten Ansichtabbilder geometrisch einfacher zueinander in Bezug gebracht werden, als dies bei alternativen Anordnungen möglich ist, was die relative Positionierung mehrerer Querschnittabbilder untereinander und das Zusammenfügen dieser Querschnittabbilder vereinfacht.

Vorzugsweise umfassen die ersten und die zweiten Bilderfassungsmittel einen gemeinsamen Bildwandler und die ersten Bilderfassungsmittel umfassen strahlumlenkende optische Elemente, wobei die strahlumlenkenden optischen Elemente so angeordnet sind, dass Lichtstrahlen zur Erzeugung des Querschnittabbilds zum gemeinsamen Bildwandler umgelenkt werden. In einer alternativen Ausführungsvariante umfassen die ersten und zweiten Bilderfassungsmittel einen gemeinsamen Bildwandler und die zweiten Bilderfassungsmittel umfassen strahlumlenkende optische Elemente, wobei die strahlumlenkenden optischen Elemente so angeordnet sind, dass Lichtstrahlen zur Erzeugung des Ansichtabbilds zum gemeinsamen Bildwandler umgelenkt werden. Beide dieser Ausführungsvarianten haben den Vorteil, dass sie nur einen Bildwandler aufweisen und somit kostengünstiger und kompakter ausgeführt werden können, als eine alternative Ausführung mit zwei separaten Bildwandlern. Die erstangeführte bevorzugte dieser beiden Ausführungsvarianten weist überdies den Vorteil auf, dass die ersten Bilderfassungsmittel auf einfache und kompakte Weise mit weiteren strahlumlenkenden optischen Elementen versehen werden können, so dass Lichtstrahlen zur Erzeugung mehrerer Querschnittabbilder aus verschiedenen Positionen zum gemeinsamen Bildwandler umgelenkt werden können.

Die ersten Bilderfassungsmittel sind vorzugsweise eingerichtet zur Erfassung und Speicherung eines zweiten Querschnittabbilds des Teilgebiets des durch den ersten Lichtprojektor beleuchteten Querschnittsteils aus einer zweiten Position ausserhalb des Strahlenbündels, gleichzeitig mit der Erfassung des ersten Querschnittabbilds, wobei die erste Position und die zweite Position auf verschiedenen Seiten einer im Strahlenbündel liegenden Ebene liegen und den beleuchteten Querschnittsteil beispielsweise unter einem gleich grossen Beobachtungswinkel erfassen. Der Vorteil, Abbilder des beleuchteten Querschnittsteils aus mehreren Positionen zu erfassen, besteht darin, dass mehrere Messwerte bestimmt und daraus durch Mittelwertbildung genauere Messresultate bestimmt werden können. Bei der Mittelwertbildung heben sich beispielsweise Abweichungen bei der Bestimmung einer ersten Distanz zwischen Augenstrukturen im ersten Querschnittabbild und einer zweiten Distanz zwischen Augenstrukturen im zweiten Querschnittabbild gegenseitig auf. Wenn folglich die opthalmologische Vorrichtung so angewendet wird, dass das Strahlenbündel im Wesentlichen senkrecht zu der dem Lichtprojektor zugewandten (Hornhaut-) Oberfläche des Auges projiziert wird, wirken sich geringe Verkippungen des Strahlenbündels bezüglich der Normalen zu der dem Lichtprojektor zugewandten Oberfläche der Hornhaut beispielsweise nicht auf die Bestimmung der Hornhautdicke aus. Auch wenn die Vorrichtung so angewendet wird, dass das Strahlenbündel im Wesentlichen entlang der optischen Achse des Auges projiziert wird, wirken sich geringe Verkippungen und Exzentrizitäten des Strahlenbündels, das heisst Verschiebungen vom Scheitelpunkt des Auges, nicht auf die Bestimmung der Hornhautdicke aus. Dasselbe trifft auf kleine Abweichungen des Beobachtungswinkels aus der ersten Position vom Beobachtungswinkels aus der zweiten Position zu. Der Vorteil, zwei Querschnittabbilder aus verschiedenen Positionen unter gleichen Beobachtungswinkeln zu erfassen, besteht also darin, dass sich kleine Ungenauigkeiten bei der Applikation, der Justierung und/oder der Kalibrierung der opthalmologischen Vorrichtung ohne grosse Abweichungen auf die Messresultate auswirken. Wird die opthalmologische Vorrichtung beispielsweise in Meridianschnitten appliziert, dann reicht eine Kalibrierung im Meridianschnitt aus, um auch bei leichten Exzentrizitäten und Verkippungen genau messen zu können. Die opthalmologische Vorrichtung ermöglicht so eine einfachere Applikation und Ausführung unter Beibehaltung der Genauigkeit der Messresultate.

In der Ausführungsvariante, in der die ersten Bilderfassungsmittel einen Bildwandler und weitere strahlumlenkende optische Elemente umfassen, sind vorzugsweise erste der strahlumlenkenden optischen Elemente so bei der ersten Position angeordnet, dass Lichtstrahlen zur Erzeugung des ersten Querschnittabbilds zum Bildwandler umgelenkt werden, und zweite der strahlumlenkenden optischen Elemente sind so bei der zweiten Position angeordnet, dass Lichtstrahlen zur Erzeugung des zweiten Querschnittabbilds zum Bildwandler umgelenkt werden. Diese ergibt eine besonders kompakte und kostengünstige Ausführung.

In einer Ausführungsvariante umfasst die opthalmologische Vorrichtung einen oder mehrere zusätzliche zweite Lichtprojektoren zur Projektion von Lichtmarken auf das Auge und die zweiten Bilderfassungsmittel sind so mit dem ersten Lichtprojektor und mit den zweiten Lichtprojektoren synchronisiert, dass bei der Erfassung und Speicherung des Ansichtabbilds des Auges, das Abbild des durch den ersten Lichtprojektor beleuchteten Querschnittsteils und ein Abbild der durch die zweiten Lichtprojektoren projizierten Lichtmarken miterfasst und mitgespeichert werden. Die projizierten und miterfassten Lichtmarken dienen als künstliche Referenzmarken, die zur Bestimmung der relativen Position der opthalmologischen Vorrichtung zum Auge und damit zur Positionsbestimmung des Querschnittabbilds respektive des beleuchteten Querschnittsteils verwendet werden können.

In einer Ausführungsvariante umfasst die opthalmologische Vorrichtung einen Schirmkörper mit einem sichtbaren Muster, welcher Schirmkörper so angeordnet ist, dass das sichtbare Muster bei der Applikation der Vorrichtung auf einer dem Auge zugewandten Seite des Schirmkörpers liegt und dass das Strahlenbündel ungehindert durch den Querschnittsteil des Auges projizierbar ist und dass das Querschnittabbild und das Ansichtabbild ungehindert durch die ersten respektive zweiten Bilderfassungsmittel erfassbar sind. Da das sichtbare Muster, beispielsweise ein Placidomuster, durch das Auge reflektiert wird, kann es mit dem Ansichtabbild erfasst werden und als künstliches Referenzmuster zur Bestimmung der relativen Position der opthalmologischen Vorrichtung zum Auge und damit zur Positionsbestimmung des Querschnittabbilds respektive des beleuchteten Querschnittsteils verwendet werden.

In einer Ausführungsvariante umfasst die opthalmologische Vorrichtung Antriebsmittel, um den ersten Lichtprojektor und die ersten und die zweiten Bilderfassungsmittel im Wesentlichen um eine Normale zu der dem ersten Lichtprojektor zugewandten Oberfläche des Auges zu rotieren oder im Wesentlichen senkrecht zu dieser Normalen zu verschieben. Durch diese Antriebsmittel wird eine automatisierte zusammenhängende Untersuchung des gesamten Auges basierend auf mehreren Querschnittabbildern ermöglicht.

Vorzugsweise ist der erste Lichtprojektor so beschaffen, dass er das Strahlenbündel in Form eines Lichtspalts projiziert. Obwohl auch andere Formen des Strahlenbündels verwendbar sind, beispielsweise punktförmig, eignet sich ein Strahlenbündel in Form eines Lichtspalts besonders für die zusammenhängende Untersuchung des gesamten Auges basierend auf mehreren Lichtschnitten des Auges.

Vorzugsweise werden die zweiten Bilderfassungsmittel so angeordnet, dass ihre optische Achse mit der optischen Achse des Auges zusammenfällt oder im Wesentlichen parallel zu der optischen Achse des Auges verläuft. Dadurch kann das Auge als Aufsichtabbild erfasst werden, was sowohl für die Positionsbestimmung des beleuchteten Querschnittsteils als auch für die Dickenbestimmung des beleuchteten Querschnittsteils vorteilhaft ist, wie bereits vorgängig erklärt wurde. In Kombination mit der bevorzugten Anordnung der zweiten Bilderfassungsmittel und des ersten Lichtprojektors, in welcher die optische Achse der zweiten Bilderfassungsmittel mit dem durch den Querschnittsteil verlaufenden Strahlenbündel zusammenfällt, ergibt sich eine Anordnung, in welcher der erste Lichtprojektor das Strahlenbündel so projiziert, dass das Strahlenbündel mit der optischen Achse des Auges zusammenfällt oder dass das Strahlenbündel parallel zu der optischen Achse des Auges verläuft.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:

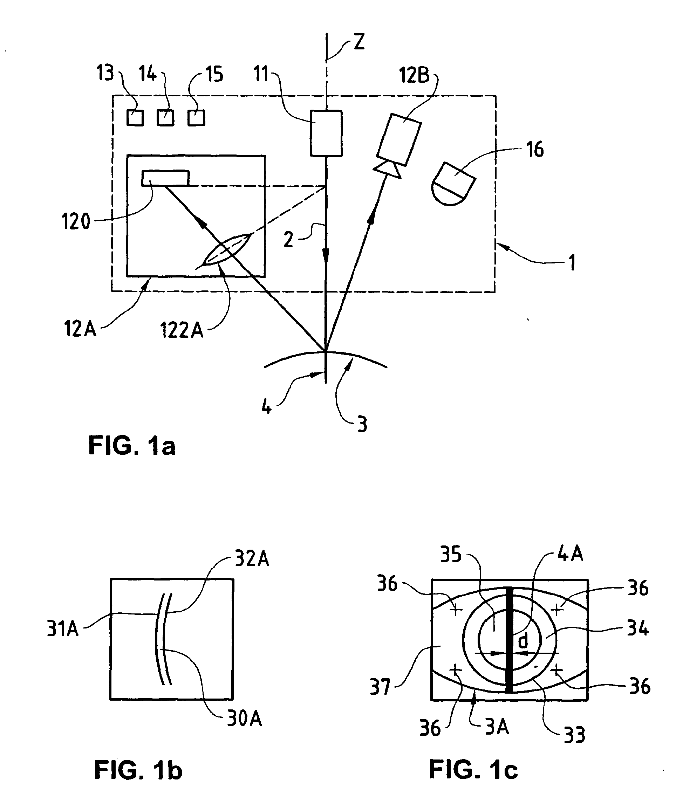

Figur 1a zeigt ein Blockdiagramm, welches schematisch eine opthalmologische Vorrichtung mit einem Lichtprojektor, einer Bilderfassungsvorrichtung zur Erfassung eines Querschnittabbilds eines Auges, einer Bilderfassungsvorrichtung zur Erfassung eines Ansichtabbilds des Auges sowie einer zusätzlichen Lichtquelle illustriert.
Figur 1b zeigt ein Querschnittabbild eines beleuchteten Querschnittsteils eines Auges (Hornhaut).
Figur 1c zeigt ein Ansichtabbild eines Auges mit einem beleuchteten Querschnittsteil.

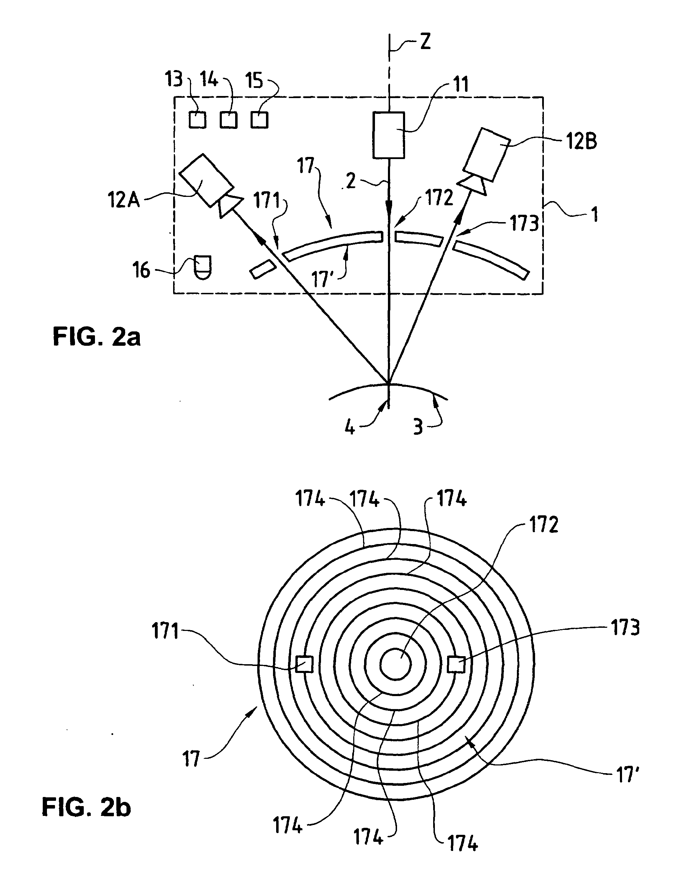

Figur 2a zeigt ein Blockdiagramm, welches schematisch eine opthalmologische Vorrichtung mit einem Lichtprojektor, einer Bilderfassungsvorrichtung zur Erfassung eines Querschnittabbilds eines Auges, einer Bilderfassungsvorrichtung zur Erfassung eines Ansichtabbilds des Auges sowie einem durchbrochenen Schirmkörper illustriert.
Figur 2b zeigt eine Ansicht der dem Auge zugewandten Seite eines Schirmkörpers mit Durchbrechungen und einem sichtbaren Muster.

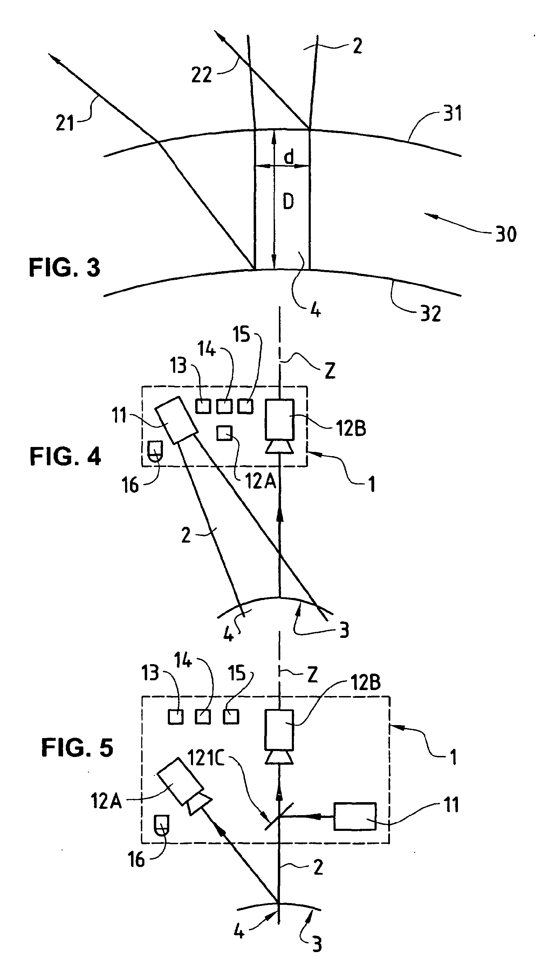

Figur 3 zeigt eine Schnittansicht eines beleuchteten Querschnittsteils eines Auges, in welcher der Strahlengang des einfallenden und reflektierten Strahlenbündels schematisch illustriert wird.
Figur 4 zeigt ein Blockdiagramm, welches schematisch eine Seitenansicht einer opthalmologische Vorrichtung mit einem Lichtprojektor und einer entlang der optischen Achse des Auges ausgerichteten Bilderfassungsvorrichtung zur Erfassung eines Ansichtabbilds eines Auges illustriert.
Figur 5 zeigt ein Blockdiagramm, welches schematisch eine opthalmologische Vorrichtung mit einem Lichtprojektor und einer Bilderfassungsvorrichtung zur Erfassung eines Ansichtabbilds eines Auges illustriert, in welcher die optische Achse der Bilderfassungsvorrichtung mit dem durch den Querschnittsteil verlaufenden Strahlenbündel zusammenfällt.

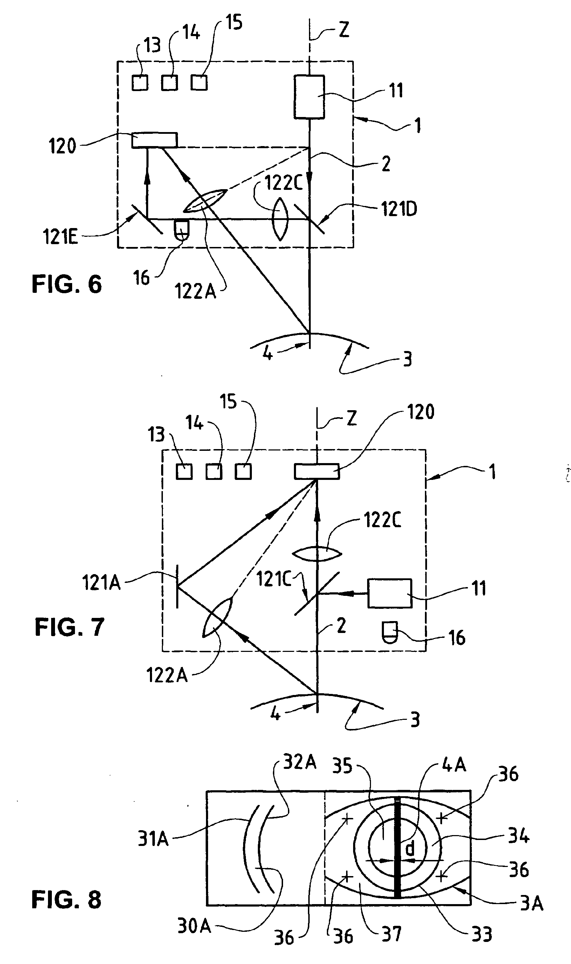

Figur 6 zeigt ein Blockdiagramm, welches schematisch eine opthalmologische Vorrichtung mit einem Lichtprojektor und Bilderfassungsmitteln zur Erfassung eines Querschnittabbilds und eines Ansichtabbilds eines Auges illustriert, in welcher Lichtstrahlen zur Erzeugung des Ansichtabbilds und Lichtstrahlen zur Erzeugung des Querschnittabbilds mittels strahlumlenkender optischer Mittel einem gemeinsamen Bildwandler zugeführt werden.
Figur 7 zeigt ein Blockdiagramm, welches schematisch eine weitere Ausführung einer opthalmologischen Vorrichtung mit einem Lichtprojektor und Bilderfassungsmitteln zur Erfassung eines Querschnittabbilds und eines Ansichtabbilds eines Auges illustriert, in welcher Lichtstrahlen zur Erzeugung des Ansichtabbilds und Lichtstrahlen zur Erzeugung des Querschnittabbilds mittels strahlumlenkender optischer Mittel einem gemeinsamen Bildwandler zugeführt werden.
Figur 8 zeigt ein kombiniertes Abbild mit einem Querschnittabbild eines beleuchteten Querschnittsteils eines Auges und einem Ansichtabbild des Auges mit dem beleuchteten Querschnittsteil.

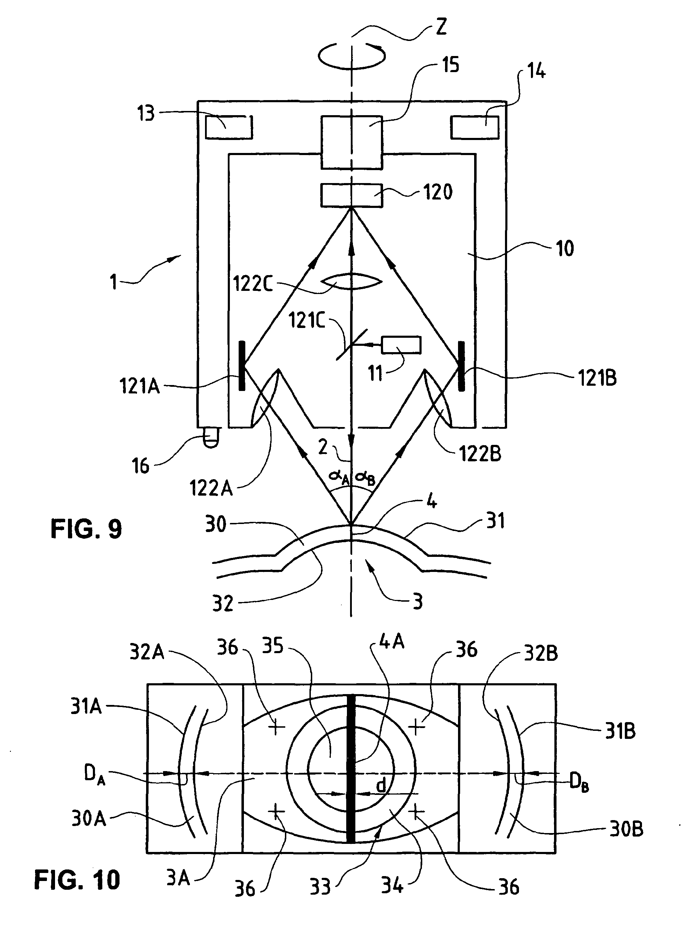

Figur 9 zeigt ein Blockdiagramm, welches schematisch eine opthalmologische Vorrichtung mit einem Lichtprojektor und Bilderfassungsmitteln zur Erfassung zweier Querschnittabbilder und eines Ansichtabbilds eines Auges illustriert, in welcher Lichtstrahlen zur Erzeugung des Ansichtabbilds und Lichtstrahlen zur Erzeugung eines ersten Querschnittabbilds aus einer ersten Position und Lichtstrahlen zur Erzeugung eines zweiten Querschnittabbilds aus einer zweiten Position mittels strahlumlenkender optischer Mittel einem gemeinsamen Bildwandler zugeführt werden.
Figur 10 zeigt ein kombiniertes Abbild mit einem ersten Querschnittabbild eines beleuchteten Querschnittsteils eines Auges aus einer ersten Position, einem Ansichtabbild des Auges mit dem beleuchteten Querschnittsteil und einem zweiten Querschnittabbild des beleuchteten Querschnittsteils aus einer zweiten Position.

### Wege zur Ausführung der Erfindung

In den Figuren 1a, 2a, 4, 5, 6, 7 und 9 bezeichnet das Bezugszeichen 1 eine opthalmologische Vorrichtung, wobei in der nachfolgenden Beschreibung mit Bezug auf diese Figuren verschiedene Ausführungsformen der opthalmologischen Vorrichtung 1 erläutert werden. Ansonsten werden in den Figuren einander entsprechende, gleiche Komponenten durch gleiche Bezugszeichen bezeichnet.

Die in den Figuren 1a, 2a, 4, 5, 6, 7 und 9 dargestellten Ausführungsformen der opthalmologischen Vorrichtung 1 umfassen einen Lichtprojektor 11 zur Projektion eines Strahlenbündels 2 durch einen Querschnittsteil eines Auges 3, insbesondere durch einen Querschnittsteil der Hornhaut 30 des Auges 3. Das Strahlenbündel 2 wird vorzugsweise in der Form eines Lichtspalts projiziert. Der Lichtprojektor 11 umfasst beispielsweise eine Spaltlampe oder einen Laser, dessen Licht durch Strahlumformungsoptiken zu einem Fächer geformt wird.

Die in den Figuren 1a, 2a, 4, 5, 6, 7 und 9 dargestellten Ausführungsformen der opthalmologischen Vorrichtung 1 umfassen Bilderfassungsmittel zur Erfassung und Speicherung eines Querschnittabbilds 30A von mindestens einem Teilgebiet des durch den Lichtprojektor 11 beleuchteten Querschnittsteils 4, welche in Scheimpfluganordnung zum Strahlenbündel 2 angeordnet sind.

Die in den Figuren 1a, 2a, 4, 5, 6, 7 und 9 dargestellten Ausführungsformen der opthalmologischen Vorrichtung 1 umfassen zudem weitere Bilderfassungsmittel zur Erfassung eines Ansichtabbilds 3A des Auges 3, welches ein Abbild des beleuchteten Querschnittsteils 4A umfasst, und zur Speicherung des erfassten Ansichtabbilds 3A und des darin enthaltenen Abbilds des beleuchteten Querschnittsteils 4A zugeordnet zum erfassten Querschnittabbild 30A.

Wie in den Figuren 1a, 2a, 4, 5, 6, 7 und 9 dargestellt ist, umfassen die Bilderfassungsmittel je nach Ausführungsform der opthalmologischen Vorrichtung 1 Bilderfassungsvorrichtungen 12A, 12B, zum Beispiel CCD-Kameras (Charged Coupled Device) oder CMOS-Kameras (Complementary Metal-Oxide-Silicon), Bildwandler 120, zum Beispiel CCD-Chips oder CMOS-Chips, strahlumlenkende optische Elemente 121A, 121B, 121E, zum Beispiel Spiegel, strahlumlenkende optische Elemente 121C, 121D, zum Beispiel strahlteilende optische Elemente wie halbdurchlässige Spiegel, und/oder abbildende optische Elemente 122A, 122B, 122C, zum Beispiel Linsen.

Zur Sichtbarmachung von natürlichen Augenmerkmalen, wie Limbus 33, Iris 34 oder Pupille 35, und/oder zur Projektion von künstlichen Lichtmarken 36 umfassen die in den Figuren 1a, 2a, 4, 5, 6, 7 und 9 dargestellten Ausführungsformen der opthalmologischen Vorrichtung 1 eine oder mehrere zusätzliche Lichtquellen 16. Insbesondere zur Sichtbarmachung natürlicher Augenmerkmale können beispielsweise eine oder mehrere Infrarotleuchtdioden verwendet werden. Die natürlichen und/oder künstlichen Referenzmerkmale werden im Ansichtabbild 3A des Auges 3 miterfasst.

Die in den Figuren 1a, 2a, 4, 5, 6, 7 und 9 dargestellten Ausführungsformen der opthalmologischen Vorrichtung 1 umfassen Verarbeitungsmittel 13 mit funktionalen Modulen zur Verarbeitung von erfassten Ansichtabbildern 3A und Querschnittabbildern 30A. Die Verarbeitungsmittel 13 umfassen mindestens einen Prozessor, Daten- und Programmspeicher. Die funktionalen Module sind vorzugsweise als programmierte Softwaremodule ausgeführt, die im Programmspeicher gespeichert sind und auf dem Prozessor ausgeführt werden. Der Fachmann wird verstehen, dass die funktionalen Module auch teilweise oder vollständig hardwaremässig ausgeführt werden können.

Die funktionalen Module der Verarbeitungsmittel 13 umfassen ein programmiertes Positionierungsmodul, welches die Position eines gespeicherten Querschnittabbilds 30A relativ zum Auge 3 bestimmt. Die relative Positionierung erfolgt auf der Basis der Ansichtabbilder 3A, die den Querschnittabbildern 30A jeweils zugeordnet sind. Die Positionsbestimmung eines Querschnittabbilds 30A erfolgt durch Positionsbestimmung des Auges 3 relativ zur opthalmologische Vorrichtung 1. Dabei wird die relative Position der opthalmologischen Vorrichtung 1 zum Auge 3 auf Grund auf Grund des Abbilds des beleuchteten Querschnittsteils 4A, der natürlichen Merkmale des Auges 3 und/oder der abgebildeten künstlichen Referenzmerkmale, beispielsweise die abgebildeten Lichtmarken 36, bestimmt. Die Position eines Querschnittabbilds 30A respektive des zugeordneten Abbilds des beleuchteten Querschnittsteils 4A kann mit Bezug zu den natürlichen Merkmalen des Auges 3 definiert werden, die im betreffenden Ansichtabbild 3A enthalten sind.

Die funktionalen Module der Verarbeitungsmittel 13 umfassen zudem ein programmiertes Kompositionsmodul, welches mehrere erfasste und gespeicherte Querschnittabbilder 30A relativ zueinander positioniert. Das Kompositionsmodul fügt unter Kenntnis der geometrischen Anordnung der opthalmologischen Vorrichtung 1 die erfassten und gespeicherten Querschnittabbilder 30A entsprechend ihrer relativen Position zum Auge 3 respektive zueinander zu einem dreidimensionalen Abbild des Auges 3 zusammen, insbesondere zu einem dreidimensionalen Abbild von Vorderkammerstrukturen des Auges 3, insbesondere der Hornhaut 30.

Die Funktions- und Ablaufsteuerung der opthalmologischen Vorrichtung 1 kann durch die Verarbeitungsmittel 13 und/oder durch weitere nicht dargestellte elektronische Steuermodule erfolgen.

Die elektrische Speisung der opthalmologischen Vorrichtung 1 erfolgt durch eine interne oder durch eine mittels Kabel angeschlossene externe Energiequelle.

Die in den Figuren 1a, 2a, 4, 5, 6, 7 und 9 dargestellten Ausführungsformen der opthalmologischen Vorrichtung 1 umfassen eine Anzeige 14, auf welcher bestimmte Messwerte und/oder Applikationshilfen angezeigt werden.

Die in den Figuren 1a, 2a, 4, 5, 6, 7 und 9 dargestellten Ausführungsformen der opthalmologischen Vorrichtung 1 umfassen Antriebsmittel 15 zum Rotieren des Lichtprojektors 11 und der Bilderfassungsmittel, im Wesentlichen um eine Normale zu der dem Lichtprojektor 11 zugewandten Oberfläche des Auges 3 oder zum Verschieben dieser Komponenten im Wesentlichen senkrecht zu dieser Normalen. Wie in der Figur 9 schematisch dargestellt ist, sind der Lichtprojektor 11 und die Bilderfassungsmittel 120, 121A, 121B, 121C, 122A, 122B, 122C zu diesem Zweck an einer beweglichen Trägervorrichtung 10 angebracht, welche durch die Antriebsmittel 15 angetrieben wird. Die zusätzliche(n) Lichtquelle(n) 16 können an der Trägervorrichtung 10 angebracht sein und mitbewegt werden oder sie können so an der opthalmologischen Vorrichtung 1 angebracht sein, dass sie nicht mit den Antriebsmitteln 15 gekoppelt sind. Die Antriebsmittel 15 umfassen vorzugsweise einen Rotationstreiber, beispielsweise ein Elektromotor, der die Trägervorrichtung 10 um die optische Achse Z des Auges rotiert. Durch die Rotation des Lichtprojektors 11 und der Bilderfassungsmittel 120, 121A, 121B, 121C, 122A, 122B, 122C um die optische Achse Z wird das gesamte Auge, insbesondere die gesamte Hornhaut 30 vermessen. In dieser Konfiguration lassen sich auf Grund der hohen Symmetrie die geringsten Messunsicherheiten erzielen.

Wie in der Figur 1a dargestellt ist, umfasst die Bilderfassungsvorrichtung 12A zur Erfassung und Speicherung von Querschnittabbildern 30A abbildende optische Elemente 122A und einen Bildwandler 120, welche in Scheimpfluganordnung zum projizierten Strahlenbündel 2 angeordnet sind. In der Figur 1b ist ein durch die Bilderfassungsvorrichtung 12A erfasstes Querschnittabbild 30A des beleuchteten Querschnittsteils 4 des Auges 3 dargestellt. Weitere Strukturen des Auges 3, wie Iris oder Linse, sind der Einfachheit halber in der Figur 1b nicht dargestellt. Im Querschnittabbild 30A sind insbesondere ein Querschnittabbild der vorderen Hornhautoberfläche 31 A und ein Querschnittabbild der hinteren Hornhautoberfläche 32A sichtbar. In der Ausführungsform gemäss Figur 1a befindet sich die optische Achse der separaten Bilderfassungsvorrichtung 12B zur Erfassung des Ansichtabbilds 3A des Auges 3 ausserhalb des Strahlenbündels 2. Das in der Figur 1c dargestellte Ansichtabbild 3A des Auges 3 entspricht allerdings einem Ansichtabbild, das durch eine Bilderfassungsvorrichtung 12B als Aufsicht erfasst wird, wobei die Bilderfassungsvorrichtung 12B so angeordnet ist, dass ihre optische Achse im Wesentlichen parallel zu der optischen Achse Z des Auges 3 oder der Sehachse des Auges 3 verläuft oder mit der optischen Achse Z oder der Sehachse des Auges 3 zusammenfällt, was beispielsweise in den Figuren 4 und 5 dargestellt ist. Im Ansichtabbild 3A sind insbesondere ein Abbild des beleuchteten Querschnittsteils 4A mit der endlichen Dicke d, die projizierten Lichtmarken 36 sowie Limbus 33, Iris 34 und Pupille 35 des Auges 3 sichtbar. Lichtmarken sind beispielsweise Glanzlichter von Leuchtdioden oder aufprojizierte Punkte. Projektionsorte sind beispielsweise die Sklera 37 oder die Hornhaut 30. Das erfasste Querschnittabbild 30A und das erfasste Ansichtabbild 3A werden den Verarbeitungsmitteln 13 zugeführt und dort im Datenspeicher einander zugeordnet gespeichert. Der Lichtprojektor 11 und die Bilderfassungsmittel 12A, 12B werden durch die Antriebsmittel 15 in weitere Aufnahmestellungen bewegt und es werden weitere Querschnittabbilder 30A und Ansichtabbilder 3A erfasst und jeweils einander zugeordnet gespeichert.

In der Figur 2a wird eine Ausführungsform der opthalmologischen Vorrichtung 1 dargestellt, welche einen durchbrochenen Schirmkörper 17 umfasst. Die Durchbrechungen 171, 172, 173 des Schirmkörpers 17 sind jeweils so angeordnet, dass die Strahlengänge zu den Bilderfassungsmitteln 12A, 12B und zum Lichtprojektor 11 den Schirmkörper 17 ungehindert passieren können. Auf der dem Auge 3 zugewandten Seite des Schirmkörpers 17 ist ein sichtbares Muster 17', ein so genanntes Placidomuster, beispielsweise mit Kreisringen 174 angebracht, das durch die Oberfläche des Auges 3 gespiegelt wird, wie beispielsweise von Keratometern bekannt. Auf dem Schirmkörper 17 können dem Auge 3 zugewandt auch Lichtquellen angebracht sein, beispielsweise Lichtprojektoren 16 zur Projektion von Lichtmarken 36. Eine Ansicht der dem Auge 3 zugewandten Seite des Schirmkörpers 17 mit Durchbrechungen 171, 172, 173 und dem sichtbaren Muster 17' ist in der Figur 2b dargestellt. Die Spiegelung des sichtbaren Musters 17' auf der Augenoberfläche wird durch die Bilderfassungsvorrichtung 12B im Ansichtabbild 3A abgebildet und kann bei der Positionierung der Querschnittabbilder 30A als künstliches Referenzmuster für die Bestimmung der relativen Position der opthalmologischen Vorrichtung 1 zum Auge 3 verwendet werden. Der Schirmkörper 17 ist vorzugsweise so mit den Antriebsmitteln 15 verbunden, dass er mit dem Lichtprojektor 11 und den Bilderfassungsmitteln mitbewegt wird. In einer alternativen Ausführung kann der Schirmkörper 17 auch so an der opthalmologischen Vorrichtung 1 angebracht sein, dass er nicht mit den Antriebsmitteln 15 gekoppelt ist mit, wobei die Durchbrechungen 171, 172, 173 entsprechend angepasst sind. An dieser Stelle soll angeführt werden, dass der Schirmkörper 17 auch so angeordnet werden kann, dass die Bilderfassungsmittel 12A, 12B und/oder der Lichtprojektor 11 zwischen den Schirmkörper 17 und das Auge 3 zu liegen kommen, dabei sind die Bilderfassungsmittel 12A, 12B und/oder der Lichtprojektor 11 beispielsweise auf der dem Auge 3 zugewandten Seite des Schirmkörpers 17 angebracht.

Die Figur 3 zeigt eine Schnittansicht eines beleuchteten Querschnittsteils 4 des Auges 3, insbesondere der Hornhaut 30. In der Figur 3 bezeichnet das Bezugszeichen 31 die vordere Hornhautoberfläche und das Bezugszeichen 32 die hintere Hornhautoberfläche. Die Hornhaut 30 wird durch das Strahlenbündel 2 im Querschnittsteil 4 beleuchtet. Wie in der Figur 3 dargestellt wird, weist das Strahlenbündel 2 eine endliche Dicke d auf. Die reflektierten Lichtstrahlen 21, 22 lassen die Dicke D des beleuchteten Querschnittsteils 4 in einem Querschnittabbild 30A auf Grund der endlichen Dicke d des Strahlenbündels dicker erscheinen als sie tatsächlich ist. Da die geometrische Anordnung von Lichtprojektor 11 und Bilderfassungsmitteln bekannt ist, kann der Einfluss der endlichen Dicke d des Strahlenbündels 2 auf das Querschnittabbild 30A respektive auf Messwerte, die aus dem Querschnittabbild 30A bestimmt werden, in Kenntnis des Werts der endlichen Dicke d korrigiert werden. Die endliche Dicke d des Strahlenbündels 2 kann besonders genau bestimmt werden, wenn das Ansichtabbild 3A durch die Bilderfassungsmittel in der Aufsicht erfasst wird.

In der Ausführungsform der opthalmologischen Vorrichtung 1 gemäss der Figur 4 ist die Bilderfassungsvorrichtung 12B so angeordnet, dass sich ein Ansichtabbild 3A erfassen lässt, das einer Aufsicht des Auges 3 entspricht. Die optische Achse der Bilderfassungsvorrichtung 12B lässt sich dafür bei der Applikation entlang der optischen Achse Z des Auges 3 ausrichten. Wie die Seitenansicht in der Figur 4 zeigt, wird das Strahlenbündel 2, das in dieser Ansicht als Lichtebene (oder Lichtfächer) dargestellt ist, in dieser Ausführungsform seitlich von ausserhalb der optischen Achse der Bilderfassungsvorrichtung 12B auf das Auge 3 projiziert.

In der Figur 5 wird eine weitere Ausführungsform der opthalmologischen Vorrichtung 1 dargestellt, welche ein Ansichtabbild 3A mit einer Aufsicht des Auges 3 ermöglicht. In der Ausführungsform gemäss Figur 5 fallen das Strahlenbündel 2 des Lichtprojektors 11, das durch den Querschnittsteil 4 verläuft, und die optische Achse der Bilderfassungsvorrichtung 12B zur Erfassung des Ansichtabbilds 3A zusammen. Dies wird beispielsweise dadurch erreicht, dass die Bilderfassungsvorrichtung 12B und der Lichtprojektor 11 so angeordnet werden, das ihre optischen Achsen in einer gemeinsamen Ebene liegen, wobei das Strahlenbündel 2 des Lichtprojektors 11 mittels des strahlumlenkenden optischen Elements 121C, auf die optische Achse der Bilderfassungsvorrichtung 12B gelenkt wird. Durch das Zusammenfallen des durch den Querschnittsteil 4 verlaufenden Strahlenbündels 2 und der optischen Achse der Bilderfassungsvorrichtung 12B wird eine besonders genaue und einfache Bestimmung der endlichen Dicke d des Strahlenbündels 2 sowie der Position der Querschnittabbilder 30A aus dem Ansichtabbild 3A ermöglicht.

In der Figur 6 wird eine weitere Ausführungsform der opthalmologischen Vorrichtung 1 dargestellt, welche ein Ansichtabbild 3A mit einer Aufsicht des Auges 3 ermöglicht und in welcher das durch den Querschnittsteil 4 verlaufende Strahlenbündel 2 und die optische Achse der Bilderfassungsmittel zur Erfassung des Ansichtabbilds 3A zusammenfallen. Die Ausführungsform gemäss der Figur 6 weist allerdings den Vorteil gegenüber derjenigen der Figur 5 auf, dass sowohl das Querschnittabbild 30A als auch das Ansichtabbild 3A mittels eines einzigen gemeinsamen Bildwandlers 120 erfasst werden. Die Ausführungsform gemäss Figur 6 ergibt sich als Weiterentwicklung der Ausführungsform gemäss Figur 1a, wobei die Lichtstrahlen zur Erzeugung des Ansichtabbilds 3A aus der Aufsicht mittels des strahlumlenkenden optischen Elements 121D, das in der optischen Achse des Lichtprojektors 11 angeordnet ist, mittels der abbildenden optischen Elemente 122C und mittels des strahlumlenkenden optischen Elements 121 E dem Bildwandler 120 zugeführt wird.

In der Figur 7 wird eine weitere Ausführungsform der opthalmologischen Vorrichtung 1 dargestellt, welche ein Ansichtabbild 3A mit einer Aufsicht des Auges 3 ermöglicht und in welcher das durch den Querschnittsteil 4 verlaufende Strahlenbündel 2 und die optische Achse der Bilderfassungsmittel zur Erfassung des Ansichtabbilds 3A zusammenfallen. Die Ausführungsform gemäss der Figur 7 weist allerdings den Vorteil gegenüber derjenigen der Figur 5 auf, dass sowohl das Querschnittabbild 30A als auch das Ansichtabbild 3A mittels eines einzigen gemeinsamen Bildwandlers 120 erfasst werden. Gegenüber der Ausführungsform gemäss der Figur 6 weist sie zudem den Vorteil auf, dass sie einfacher und kompakter ausführbar ist. Die Ausführungsform gemäss Figur 7 ergibt sich als Weiterentwicklung der Ausführungsform gemäss Figur 5, wobei die Lichtstrahlen zur Erzeugung des Querschnittabbilds 30A mittels der abbildenden optischen Elemente 122A, das mit dem Bildwandler 120 in Scheimpfluganordnung zum durch den Querschnittsteil 4 verlaufenden Strahlenbündel 2 angeordnet ist, und mittels des strahlumlenkenden optischen Elements121A dem Bildwandler 120 zugeführt wird. Die in der Figur 5 dargestellte Bilderfassungsvorrichtung 12B entspricht der Kombination des Bildwandlers 120 und der abbildenden optischen Elemente 122C der Figur 7.

In der Figur 8 ist das kombinierte Querschnittabbild 30A und Ansichtabbild 3A dargestellt, das durch den Bildwandler 120 der Ausführungsformen gemäss den Figuren 6 und 7 erfasst wird. Das kombinierte Abbild kann wie in der Figur 8 dargestellt erzeugt werden, indem das Querschnittabbild 30A und das Ansichtsabbild 3A nebeneinander getrennt erfasst werden. Das Querschnittabbild 30A und das Ansichtsabbild 3A können unter Zuhilfenahme von Farbfiltern, die beispielsweise von Farbkameras bekannt sind, und mehreren Lichtquellen mit unterschiedlichen Farben jedoch auch teilweise oder vollständig überlagert erfasst werden. Eine Bildtrennung wird dann von den Verarbeitungsmitteln 13 anhand der Farben durchgeführt. Es ist auch möglich, optische oder elektrische Verschlüsse (so genannte Shutters) zu verwenden und das Querschnittabbild 30A und das Ansichtabbild 3A schnell hintereinander als getrennte Abbilder zu erfassen, so dass Relativbewegungen zwischen Auge 3 und opthalmologischer Vorrichtung 1 keinen merklichen Einfluss haben. Bei der Verwendung von Kameras respektive Bildwandlern mit Halbbildern und gepulsten Lichtquellen können das Querschnittabbild 30A und das Ansichtabbild 3A hintereinander synchronisiert als zwei Halbbilder erfasst werden.

In der Figur 9 wird eine weitere Ausführungsform der opthalmologischen Vorrichtung 1 dargestellt, welche ein Ansichtabbild 3A mit einer Aufsicht des Auges 3 ermöglicht und in welcher das durch den Querschnittsteil 4 verlaufende Strahlenbündel 2 und die optische Achse der Bilderfassungsmittel zur Erfassung des Ansichtabbilds 3A zusammenfallen. Die Ausführungsform gemäss Figur 9 ergibt sich als Weiterentwicklung der Ausführungsform gemäss Figur 7, wobei die opthalmologische Vorrichtung 1 mit weiteren abbildenden optischen Elementen 122B und einem weiteren strahlumlenkenden optischen Element 121B versehen ist, um zusätzlich ein zweites Querschnittabbild 30B zu erfassen. Die abbildenden optischen Elemente 122A und das strahlumlenkende optische Element 121A leiten die Lichtstrahlen zur Erfassung des Querschnittabbilds 30A aus einer ersten Position unter dem Beobachtungswinkel α_{A} zur Erfassung an den Bildwandler 120. Die zusätzlichen abbildenden optischen Elemente 122B und das zusätzliche strahlumlenkende optische Element 121B leiten die Lichtstrahlen zur Erfassung des Querschnittabbilds 30B aus einer zweiten Position unter dem Beobachtungswinkel α_{B} zur Erfassung ebenfalls an den Bildwandler 120. Vorzugsweise befinden sich die beiden Positionen auf verschiedenen Seiten des Strahlenbündels 2 und die Beträge der Beobachtungswinkel α_{A} und α_{B} sind vorzugsweise gleich gross. Gegenüber der Ausführungsform gemäss der Figur 7 weist die Ausführungsform gemäss der Figur 9 den Vorteil auf, dass Messresultate, die aus Messwerten am Querschnittabbild 30A bestimmt werden in der opthalmologischen Vorrichtung 1 gemäss der Figur 9 aus zwei Messwerten von zwei Querschnittabbildern 30A und 30B, die aus verschiedenen Positionen erfasst wurden, durch Mittelwertbildung genauer bestimmt werden können. Zum Beispiel kann die Hornhautdicke D durch Mittelwertbildung aus den Messwerten D_{A} und D_{B} genauer bestimmt werden, wie in der zum Anmeldezeitpunkt unveröffentlichten europäischen Patentanmeldung Nr. 02405272 beschrieben wurde.

In der Figur 10 wird das kombinierte Querschnittabbild 30A, Ansichtabbild 3A und Querschnittabbild 30B dargestellt, das durch den Bildwandler 120 der Ausführungsformen gemäss der Figur 9 erfasst wird. Das kombinierte Abbild kann wie in der Figur 10 dargestellt erzeugt werden, indem das Querschnittabbild 30A, das Ansichtsabbild 3A und das Querschnittabbild 30B nebeneinander getrennt erfasst werden. Das kombinierte Abbild kann jedoch auch anders erfasst und dargestellt werden, wie im Zusammenhang mit Figur 8 bereits erwähnt wurde. Zudem kann der Fachmann andere Bildanordnungen vornehmen und beispielsweise die beiden Querschnittabbilder 30A, 30B direkt nebeneinander über dem Ansichtabbild 3A darstellen.

Die funktionalen Module der Verarbeitungsmittel 13 umfassen zudem auch programmierte Auswertungsmodule, beispielsweise Messmodule, welche in den erfassten und gespeicherten Querschnittabbildern 30A, 30B Augenstrukturen bestimmen, insbesondere Abbilder der Hornhaut mit der vorderen Hornhautoberfläche 31 A, 31 B und der hinteren Hornhautoberfläche 32A, 32B, und darauf basierend Distanzen respektive Dicken bestimmen, insbesondere die Messwerte D_{A} und D_{B} der Distanzen zwischen der vorderen Hornhautoberfläche 31A, 31 B und der hinteren Hornhautoberfläche 32A, 32B zur Bestimmung der Hornhautdicke D.

Abschliessend soll angemerkt werden, dass die opthalmologische Vorrichtung 1 vorzugsweise als kompakte Messprobe ausgeführt ist, wobei zusätzliche Verarbeitungsmittel für die umfassende Erfassung des gesamten Auges 3 in einer externen Verarbeitungseinheit, beispielsweise in einem Personal Computer, ausgeführt sein können, wobei der Datenaustausch über eine kontaktbehaftete oder kontaktlose Kommunikationsverbindung erfolgt. Bestimmte Messresultate, beispielsweise die lokale Hornhautdicke D oder - -topografie, können auf der Anzeige 14 oder auf einer Anzeige der externen Verarbeitungseinheit angezeigt werden.

## Patentansprüche

1. Ophthalmologische Vorrichtung (1), umfassend:
- einen ersten Lichtprojektor (11) zur Projektion eines Strahlenbündels (2) durch einen Querschnittsteil (4) eines Auges (3), insbesondere durch einen Querschnittsteil (4) der Hornhaut des Auges (3),
- erste Bilderfassungsmittel zur Erfassung eines Querschnittabbilds (30A) von mindestens einem Teilgebiet des durch den ersten Lichtprojektor (11) beleuchteten Querschnittsteils (4), aus einer ersten Position ausserhalb des Strahlenbündels (2), welche in Scheimpfluganordnung zum Strahlenbündel (2) angeordnet sind,
- zweite Bilderfassungsmittel zur Erfassung eines Ansichtabbilds (3A) des Auges (3) derart, dass das Ansichtabbild (3A) ein Abbild (4A) des durch den ersten Lichtprojektor (11) beleuchteten Querschnittsteils (4) umfasst,
**gekennzeichnet durch**
- Verarbeitungsmittel (13) zur Speicherung des erfassten Querschnittabbilds (30A) und des erfassten Ansichtabbilds (3A) einander zugeordnet in einem Datenspeicher, und zur Bestimmung der Position des gespeicherten Querschnittabbilds (30A) relativ zum Auge auf der Basis des zugeordnet gespeicherten Ansichtabbilds (3A).

2. Vorrichtung (1) gemäss Anspruch 1, **dadurch gekennzeichnet, dass** die Verarbeitungsmittel (13) eingerichtet sind zur Positionierung des gespeicherten Querschnittabbilds (30A) auf der Basis des zugeordnet gespeicherten Ansichtabbilds (3A) relativ zu vorgängig gespeicherten Querschnittabbildem des Auges (3).

3. Vorrichtung (1) gemäss einem der Ansprüche 1 oder 2, **dadurch gekennzeichnet, dass** die Verarbeitungsmittel (13) eingerichtet sind zum Bestimmen der Dicke (d) des durch den ersten Lichtprojektor (11) beleuchteten Querschnittsteils (4) des Auges (3) auf der Basis des gespeicherten Ansichtabbilds (3A).

4. Vorrichtung (1) gemäss einem der Ansprüche 1 bis 3, **dadurch gekennzeichnet, dass** die zweiten Bilderfassungsmittel und der erste Lichtprojektor (11) so angeordnet sind, dass die optische Achse der zweiten Bilderfassungsmittel mit dem durch den Querschnittsteil (4) verlaufenden Strahlenbündel (2) zusammenfällt.

5. Vorrichtung (1) gemäss einem der Ansprüche 1 bis 4, **dadurch gekennzeichnet, dass** die ersten und die zweiten Bilderfassungsmittel jeweils so angeordnet sind, dass ihre optischen Achsen in einer gemeinsamen Ebene liegen.

6. Vorrichtung (1) gemäss einem der Ansprüche 1 bis 5, **dadurch gekennzeichnet, dass** die ersten und die zweiten Bilderfassungsmittel einen gemeinsamen Bildwandler (120) umfassen, und dass die ersten Bilderfassungsmittel strahlumlenkende optische Elemente (121A) umfassen, wobei die strahlumlenkenden optischen Elemente (121A) so angeordnet sind, dass Lichtstrahlen zur Erzeugung des Querschnittabbilds (30A) zum gemeinsamen Bildwandler (120) umgelenkt werden.

7. Vorrichtung (1) gemäss einem der Ansprüche 1 bis 5, **dadurch gekennzeichnet, dass** die ersten und zweiten Bilderfassungsmittel einen gemeinsamen Bildwandler (120) umfassen, und dass die zweiten Bilderfassungsmittel strahlumlenkende optische Elemente (121 E) umfassen, wobei die strahlumlenkenden optischen Elemente (121 E) so angeordnet sind, dass Lichtstrahlen zur Erzeugung des Ansichtabbilds (3A) zum gemeinsamen Bildwandler (120) umgelenkt werden.

8. Vorrichtung (1) gemäss einem der Ansprüche 1 bis 7, **dadurch gekennzeichnet, dass** die ersten Bilderfassungsmittel eingerichtet sind zur Erfassung und Speicherung eines zweiten Querschnittabbilds (30B) des Teilgebiets des durch den ersten Lichtprojektor (11) beleuchteten Querschnittsteils (4) aus einer zweiten Position ausserhalb des Strahlenbündels (2), gleichzeitig mit der Erfassung des ersten Querschnittabbilds (30A), wobei die erste Position und die zweite Position auf verschiedenen Seiten einer im Strahlenbündel (2) liegenden Ebene liegen.

9. Vorrichtung (1) gemäss Anspruch 8, **dadurch gekennzeichnet, dass** die ersten Bilderfassungsmittel einen Bildwandler (120) umfassen, und dass die ersten Bilderfassungsmittel strahlumlenkende optische Elemente (121A, 121B) umfassen, wobei erste der strahlumlenkenden optischen Elemente (121A) so bei der ersten Position angeordnet sind, dass Lichtstrahlen zur Erzeugung des ersten Querschnittabbilds (30A) zum Bildwandler (120) umgelenkt werden und wobei zweite der strahlumlenkenden optischen Elemente (121 B) so bei der zweiten Position angeordnet sind, dass Lichtstrahlen zur Erzeugung des zweiten Querschnittabbilds (30B) zum Bildwandler (120) umgelenkt werden.

10. Vorrichtung (1) gemäss einem der Ansprüche 1 bis 9, **dadurch gekennzeichnet, dass** sie einen oder mehrere zusätzliche zweite Lichtprojektoren (16) zur Projektion von Lichtmarken (36) auf das Auge (3) umfasst, und dass die zweiten Bilderfassungsmittel so mit dem ersten Lichtprojektor (11) und mit den zweiten Lichtprojektoren (16) synchronisiert sind, dass bei der Erfassung und Speicherung des Ansichtabbilds (3A) des Auges (3), das Abbild (4A) des durch den ersten Lichtprojektor (11) beleuchteten Querschnittsteils (4) und ein Abbild der durch die zweiten Lichtprojektoren (16) projizierten Lichtmarken (36) miterfasst und mitgespeichert werden.

11. Vorrichtung (1) gemäss einem der Ansprüche 1 bis 10, **dadurch gekennzeichnet, dass** sie einen Schirmkörper (17) mit einem sichtbaren Muster (17') umfasst, welcher Schirmkörper (17) so angeordnet ist, dass das sichtbare Muster (17') bei der Applikation der Vorrichtung (1) auf einer dem Auge (3) zugewandten Seite des Schirmkörpers (17) liegt, und wobei der Schirmkörper (17) so angeordnet ist, dass das Strahlenbündel (2) ungehindert durch den Querschnittsteil (4) des Auges (3) projizierbar ist und dass das Querschnittabbild (30A) und das Ansichtabbild (3A) ungehindert durch die ersten respektive zweiten Bilderfassungsmittel erfassbar sind.

12. Vorrichtung (1) gemäss einem der Ansprüche 1 bis 11, **dadurch gekennzeichnet, dass** sie Antriebsmittel (15) umfasst, um den ersten Lichtprojektor (11) und die ersten und die zweiten Bilderfassungsmittel im Wesentlichen um eine Normale zu der dem ersten Lichtprojektor (11) zugewandten Oberfläche des Auges (3) zu rotieren oder im Wesentlichen senkrecht zu dieser Normalen zu verschieben.

13. Vorrichtung (1) gemäss einem der Ansprüche 1 bis 12, **dadurch gekennzeichnet, dass** der erste Lichtprojektor (11) so beschaffen ist, dass er das Strahlenbündel (2) in Form eines Lichtspalts projiziert.

14. Ophthalmologisches Messverfahren, umfassend:
- Projektion eines Strahlenbündels (2) durch einen Querschnittsteil (4) eines Auges (3), insbesondere durch einen Querschnittsteil (4) der Hornhaut des Auges (3), mittels eines ersten Lichtprojektors (11),
- Erfassung eines Querschnittabbilds (30A) von mindestens einem Teilgebiet des durch den ersten Lichtprojektor (11) beleuchteten Querschnittsteils (4), aus einer ersten Position ausserhalb des Strahlenbündels (2), mittels erster Bilderfassungsmittel, die in Scheimpfluganordnung zum Strahlenbündel (2) angeordnet werden,
- Erfassung eines Ansichtabbilds (3A) des Auges (3) mittels zweiter Bilderfassungsmittel derart, dass das Ansichtabbild (3A) ein Abbild (4A) des durch den ersten Lichtprojektor (11) beleuchteten Querschnittsteils (4) umfasst,
**gekennzeichnet durch**
- Speicherung des erfassten Querschnittabbilds (30A) und des erfassten Ansichtabbilds (3A) einander zugeordnet in einem Datenspeicher, und
- Bestimmung der Position des gespeicherten Querschnittabbilds (30A) relativ zum Auge (3) auf der Basis des zugeordnet gespeicherten Ansichtabbilds (3A).

15. Verfahren gemäss Anspruch 14, **dadurch gekennzeichnet, dass** das gespeicherte Querschnittabbild (30A) auf der Basis des zugeordnet gespeicherten Ansichtabbilds (3A) relativ zu vorgängig gespeicherten Querschnittabbildern des Auges (3) positioniert wird.

16. Verfahren gemäss einem der Ansprüche 14 oder 15, **dadurch gekennzeichnet, dass** die Dicke (d) des durch den ersten Lichtprojektor (11) beleuchteten Querschnittsteils (4) des Auges (3) auf der Basis des gespeicherten Ansichtabbilds (3A) bestimmt wird.

17. Verfahren gemäss einem der Ansprüche 14 bis 16, **dadurch gekennzeichnet, dass** die zweiten Bilderfassungsmittel und der erste Lichtprojektor (11) so angeordnet werden, dass die optische Achse der zweiten Bilderfassungsmittel mit dem durch den Querschnittsteil (4) verlaufenden Strahlenbündel (2) zusammenfällt.

18. Verfahren gemäss einem der Ansprüche 14 bis 17, **dadurch gekennzeichnet, dass** die ersten Bilderfassungsmittel und die zweiten Bilderfassungsmittel so angeordnet werden, dass ihre optischen Achsen in einer gemeinsamen Ebene liegen.

19. Verfahren gemäss einem der Ansprüche 14 bis 18, **dadurch gekennzeichnet, dass** die ersten Bilderfassungsmittel mit strahlumlenkenden optischen Elementen (121A) versehen werden, wobei die strahlumlenkenden optischen Elemente (121A) so angeordnet werden, dass Lichtstrahlen zur Erzeugung des Querschnittabbilds (30A) zu einem mit den zweiten Bilderfassungsmitteln gemeinsam verwendeten Bildwandler (120) umgelenkt werden.

20. Verfahren gemäss einem der Ansprüche 14 bis 18, **dadurch gekennzeichnet, dass** die zweiten Bilderfassungsmittel mit strahlumlenkenden optischen Elementen (121E) versehen werden, wobei die strahlumlenkenden optischen Elemente (121 E) so angeordnet werden, dass Lichtstrahlen zur Erzeugung des Ansichtabbilds (3A) zu einem mit den ersten Bilderfassungsmitteln gemeinsam verwendeten Bildwandler (120) umgelenkt werden.

21. Verfahren gemäss einem der Ansprüche 14 bis 20, **dadurch gekennzeichnet, dass** gleichzeitig mit der Erfassung des ersten Querschnittabbilds (30A) ein zweites Querschnittabbild (30B) des Teilgebiets des durch den ersten Lichtprojektor (11) beleuchteten Querschnittsteils (4) mittels der ersten Bilderfassungsmittel aus einer zweiten Position ausserhalb des Strahlenbündels (2) erfasst und gespeichert wird, wobei die erste Position und die zweite Position auf verschiedenen Seiten einer im Strahlenbündel (2) liegenden Ebene festgelegt werden.

22. Verfahren gemäss Anspruch 21, **dadurch gekennzeichnet, dass** erste strahlumlenkende optische Elemente (121 A) der ersten Bilderfassungsmittel so bei der ersten Position angeordnet werden, dass sie Lichtstrahlen zur Erzeugung des ersten Querschnittabbilds (30A) zu einem Bildwandler (120) der ersten Bilderfassungsmittel umlenken, und dass zweite strahlumlenkende optische Elemente (121 B) der ersten Bilderfassungsmittel so bei der zweiten Position angeordnet werden, dass sie Lichtstrahlen zur Erzeugung des zweiten Querschnittabbilds (30B) zum Bildwandler (120) der ersten Bilderfassungsmittel umlenken.

23. Verfahren gemäss einem der Ansprüche 14 bis 22, **dadurch gekennzeichnet, dass** Lichtmarken (36) mittels eines oder mehrerer zusätzlichen zweiten Lichtprojektoren (16) auf das Auge (3) projiziert werden, und dass die zweiten Bilderfassungsmittel so mit dem ersten Lichtprojektor (11) und mit den zweiten Lichtprojektoren (16) synchronisiert werden, dass bei der Erfassung und Speicherung des Ansichtabbilds (3A) des Auges (3), das Abbild (4A) des durch den ersten Lichtprojektor (11) beleuchteten Querschnittsteils (4) und ein Abbild der durch die zweiten Lichtprojektoren (16) projizierten Lichtmarken (36) miterfasst und mitgespeichert werden.

24. Verfahren gemäss einem der Ansprüche 14 bis 23, **dadurch gekennzeichnet, dass** ein mit einem sichtbaren Muster (17') versehener Schirmkörper (17) so angeordnet wird, dass das sichtbare Muster (17') dem Auge (3) zugewandt wird und dass das Strahlenbündel (2) ungehindert durch den Querschnittsteil (4) des Auges (3) projiziert wird und dass das Querschnittabbild (30A) und das Ansichtabbild (3A) ungehindert durch die ersten respektive zweiten Bilderfassungsmittel erfasst werden.

25. Verfahren gemäss einem der Ansprüche 14 bis 24, **dadurch gekennzeichnet, dass** der erste Lichtprojektor (11) und die ersten und die zweiten Bilderfassungsmittel im Wesentlichen um eine Normale zu der dem ersten Lichtprojektor (11) zugewandten Oberfläche des Auges (3) rotiert werden oder im Wesentlichen senkrecht zu dieser Normalen verschoben werden.

26. Verfahren gemäss einem der Ansprüche 14 bis 25, **dadurch gekennzeichnet, dass** der erste Lichtprojektor (11) das Strahlenbündel (2) in Form eines Lichtspalts projiziert.

27. Verfahren gemäss einem der Ansprüche 14 bis 26, **dadurch gekennzeichnet, dass** die zweiten Bilderfassungsmittel so angeordnet werden, dass ihre optische Achse mit der optischen Achse (Z) des Auges (3) zusammenfällt oder im Wesentlichen parallel zu der optischen Achse (Z) des Auges (3) verläuft.

28. Verfahren gemäss einem der Ansprüche 14 bis 26, **dadurch gekennzeichnet, dass** die zweiten Bilderfassungsmittel so angeordnet werden, dass ihre optische Achse mit der Sehachse des Auges (3) zusammenfällt oder im Wesentlichen parallel zu der Sehachse des Auges (3) verläuft.

## Claims

1. Ophthalmologic device (1), comprising:
- a first light projector (11) for projection of a beam of rays (2) through a cross-sectional portion (4) of an eye (3), in particular through a cross-sectional portion (4) of the cornea of the eye (3),
- first image-capturing means for capturing a cross-sectional image (30A) of at least one sub-area of the cross-sectional portion (4), illuminated by the first light projector (11), from a first position outside the beam of rays (2), which means are disposed in Scheimpflug configuration with respect to the beam of rays (2), and
- second image-capturing means for capturing a view image (3A) of the eye (3), in such a way that the view image (3A) comprises an image (4A) of the cross-sectional portion (4) illuminated by the first light projector (11),
**characterised by**
- processing means (13) for storing the captured cross-sectional image (30A) and the captured view image (3A) assigned to each other in a data store, and determining the position of the stored cross-sectional image (30A) relative to the eye on the basis of the stored assigned view image (3A).

2. Device (1) according to claim 1, wherein the processing means (13) are designed to position the stored cross-sectional image (30A) relative to the previously stored cross-sectional images of the eye (3) on the basis of the stored assigned view image (3A).

3. Device (1) according to one of claims 1 or 2, wherein the processing means (13) are set up to determine the thickness (d) of the cross-sectional portion (4), illuminated by the first light projector, of the eye (3) on the basis of the stored view image (3A).

4. Device (1) according to one of claims 1 to 3, wherein the second image-capturing means and the first light projector (11) are disposed such that the optic axis of the second image-capturing means coincides with the beam of rays (2) running through the cross-sectional portion (4).

5. Device (1) according to one of claims 1 to 4, wherein the first and the second image-capturing means are disposed such that their optic axes are situated in a common plane.

6. Device (1) according to one of claims 1 to 5, wherein the first and the second image-capturing means comprise a common image converter (120), and the first image-capturing means comprise ray-redirecting optical elements (121A), the ray-redirecting optical elements (121A) being disposed such that, for generation of the cross-sectional image (30A), light beams are redirected to the common image converter (120).

7. Device (1) according to one of claims 1 to 5, wherein the first and second image-capturing means comprise a common image converter (120), and the second image-capturing means comprise ray-redirecting optical elements (121 E), the ray-redirecting optical elements (121 E) being disposed such that light beams are redirected to the common image converter (120) for generation of the view image (3A).

8. Device (1) according to one of claims 1 to 7, wherein the first image-capturing means are set up to capture and store a second cross-sectional image (30B) of the sub-area of the cross-sectional portion (4) illuminated by the first light projector (11), from a second position outside the beam of rays (2) simultaneously with the capturing of the first cross-sectional image (30A), the first position and the second position lying on different sides of a plane situated in the beam of rays (2).

9. Device (1) according to claim 8, wherein the first image-capturing means comprise an image converter (120), and the first image-capturing means comprise ray-redirecting optical elements (121A, 121 B), the first of the ray-redirecting optical elements (121A) being disposed at the first position in such a way that light beams are redirected to the image converter (120) for generation of a first cross-sectional image (30A), and second of the ray-redirecting optical elements (121 B) being disposed at the second position in such a way that light beams are redirected to the image converter (120) for generation of the second cross-sectional image (30B).

10. Device (1) according to one of claims 1 to 9, wherein it comprises one or more additional second light projectors (16) for projection of light markers (36) on the eye (3), and the second image-capturing means are synchronized with the first light projector (11) and with the second light projectors (16) in such a way that, during the capturing and storing of the view image (3A) of the eye (3), the image of the cross-sectional portion (4) illuminated by the first light projector (11) and an image of the light markers (36) projected by the second light projectors (16) are co-captured and co-stored.

11. Device (1) according to one of claims 1 to 10, wherein it comprises a screen element (17) with a visible pattern (17'), which screen element (17) is disposed such that the visible pattern (17') is situated on a side of the screen element (17) turned toward the eye (3) during application of the device, and the screen element (17) being disposed such that the beam of rays (2) is able to be projected unimpeded through the cross-sectional portion (4) of the eye (3), and such that the cross-sectional image (30A) and the view image (3A) are able to be captured unimpeded by the first, respectively second, image-capturing means.

12. Device (1) according to one of claims 1 to 11, wherein it comprises drive means (15) to rotate the first light projector (11) and the first and the second image-capturing means substantially about a normal to the surface of the eye (3) turned toward the first light projector (11) or to shift them substantially perpendicular to this normal.

13. Device (1) according to one of claims 1 to 12, wherein the first light projector (11) is designed such that it projects the beam of rays (2) in the form of a light slit.

14. Ophthalmologic measuring method, comprising:
- projecting a beam of rays (2) through a cross-sectional portion (4) of an eye (3), in particular through a cross-sectional portion (4) of the cornea of the eye (3), by means of a first light projector (11),
- capturing a cross-sectional image (30A) of at least one sub-area of the cross-sectional portion (4), illuminated by the first light projector (11), from a first position outside the beam of rays (2), by means of first image-capturing means which are disposed in Scheimpflug configuration with respect to the beam of rays (2),
- capturing a view image (3A) of the eye (3) by means of second image-capturing means, in such a way that the view image (3A) comprises an image (4A) of the cross-sectional portion (4) illuminated by the first light projector (11),
**characterised by**
- storing the captured cross-sectional image (30A) and the captured view image (3A) assigned to each other in a data store, and
- determining the position of the stored cross-sectional image (30A) relative to the eye (3) on the basis of the stored assigned view image (3A).

15. Method according to claim 14, wherein the stored cross-sectional image (30A) is positioned relative to the previously stored cross-sectional images of the eye (3) on the basis of the stored assigned view image (3A).

16. Method according to one of claims 14 or 15, wherein the thickness (d) of the cross-sectional portion (4) of the eye (3) illuminated by the first light projector (11) is determined on the basis of the stored view image (3A).

17. Method according to one of claims 14 to 16, wherein the second image-capturing means and the first light projector (11) are disposed such that the optic axis of the second image-capturing means coincides with the beam of rays (2) running through the cross-sectional portion (4).

18. Method according to one of claims 14 to 17, wherein the first image-capturing means and the second image-capturing means are disposed such that their optic axes lie in a common plane.

19. Method according to one of claims 14 to 18, wherein the first image-capturing means are provided with ray-redirecting optical elements (121A), the ray-redirecting optical elements (121A) being disposed such that for generation of the cross-sectional image (30A) light beams are redirected to an image converter (120) used jointly with the second image-capturing means.

20. Method according to one of claims 14 to 18, wherein the second image-capturing means are provided with ray-redirecting optical elements (121 E), the ray-redirecting optical elements (121 E) being disposed such that for generation of the view image (3A) light beams are redirected to an image converter (120) used jointly with the first image-capturing means.

21. Method according to one of claims 14 to 20, wherein simultaneously with the capturing of the first cross-sectional image (30A) a second cross-sectional image (30B) is captured of the sub-area of the cross-sectional portion (4), illuminated by the first light projector (11), by means of the first image-capturing means from a second position outside the beam of rays (2) and is stored, the first position and the second position being established on different sides of a plane situated in the beam of rays (2).

22. Method according to claim 21, wherein first ray-redirecting optical elements (121A) of the first image-capturing means are disposed at the first position in such a way that for generating the first cross-sectional image (30A) they redirect light beams to an image converter (120) of the first image-capturing means, and second ray-redirecting optical elements (121 B) of the first image-capturing means are disposed at the second position in such a way that for generating the second cross-sectional image (30B) they redirect light beams to the image converter (120) of the first image-capturing means.

23. Method according to one of claims 14 to 22, wherein light markers (36) are projected on the eye (3) by means of one or more additional second light projectors (16), and the second image-capturing means are synchronized with the first light projector (11) and with the second light projectors (16) in such a way that during the capturing and storing of the view image (3A) of the eye (3), the image of the cross-sectional portion (4) illuminated by the first light projector (11) and an image of the light markers (36) projected by the second light projectors (16) are co-captured and co-stored.

24. Method according to one of claims 14 to 23, wherein a screen element (17) provided with a visible pattern (17') is disposed such that the visible pattern (17') is turned toward the eye (3), and the beam of rays (2) is projected unimpeded through the cross-sectional portion (4) of the eye (3), and the cross-sectional image (30A) and the view image (3A) are captured by the first, respectively second, image-capturing means in an unimpeded way.

25. Method according to one of claims 14 to 24, wherein the first light projector (11) and the first and the second image-capturing means are rotated substantially about a normal to the surface of the eye (3) turned toward the first light projector (11) or are shifted substantially perpendicular to this normal.

26. Method according to one of claims 14 to 25, wherein the first light projector (11) projects the beam of rays (2) in the form of a light slit.

27. Method according to one of claims 14 to 26, wherein the second image-capturing means are disposed such that their optic axis coincides with the optic axis (Z) of the eye (3) or runs substantially parallel to the optic axis (Z) of the eye (3).

28. Method according to one of claims 14 to 26, wherein the second image-capturing means are disposed such that their optic axis coincides with the line of vision of the eye (3) or runs substantially parallel to the line of vision of the eye (3).

## Revendications

1. Dispositif ophtalmologique (1), comprenant :
- un premier projecteur de lumière (11) pour projeter un faisceau de rayons (2) à travers une partie en section transversale (4) d'un oeil (3), notamment à travers une partie en section transversale (4) de la cornée de l'oeil (3),
- des premiers moyens de détection d'image pour détecter une image en section transversale (30A) d'au moins une région partielle de la partie en section transversale (4) éclairée par le premier projecteur de lumière (11), à partir d'une première position en dehors du faisceau de rayons (2), qui sont disposés suivant un système Scheimpflug par rapport au faisceau de rayons (2),
- des deuxièmes moyens de détection d'image pour détecter une image en projection (3A) de l'oeil (3), de telle sorte que l'image en projection (3A) comprenne une image (4A) de la partie en section transversale (4) éclairée par le premier projecteur de lumière (11),
**caractérisé par**
- des moyens de traitement (13) pour mémoriser l'image en section transversale (30A) détectée et l'image en projection (3A) détectée de manière associée mutuellement dans une mémoire de données, et pour déterminer la position de l'image en section transversale (30A) mémorisée par rapport à l'oeil sur la base de l'image en projection (3A) mémorisée de manière associée.

2. Dispositif (1) selon la revendication 1, **caractérisé en ce que** les moyens de traitement (13) pour prévus pour positionner l'image en section transversale (30A) mémorisée sur la base de l'image en projection (3A) mémorisée, par rapport à des images en section transversale de l'oeil (3) préalablement mémorisées.

3. Dispositif (1) selon l'une quelconque des revendications 1 ou 2, **caractérisé en ce que** les moyens de traitement (13) sont prévus pour déterminer l'épaisseur (d) de la partie en section transversale (4) de l'oeil (3) éclairée par le premier projecteur de lumière (11) sur la base de l'image en projection (3A) mémorisée.

4. Dispositif (1) selon l'une quelconque des revendications 1 à 3, **caractérisé en ce que** les deuxièmes moyens de détection d'image et le premier projecteur de lumière (11) sont disposés de telle sorte que l'axe optique des deuxièmes moyens de détection d'image coïncide avec le faisceau de rayons (2) s'étendant à travers la partie en section transversale (4).

5. Dispositif (1) selon l'une quelconque des revendications 1 à 4, **caractérisé en ce que** les premiers et deuxièmes moyens de détection d'image sont disposés à chaque fois de telle sorte que leurs axes optiques se situent dans un plan commun.

6. Dispositif (1) selon l'une quelconque des revendications 1 à 5, **caractérisé en ce que** les premiers et deuxièmes moyens de détection d'image présentent un convertisseur d'image commun (120), et **en ce que** les premiers moyens de détection d'image comprennent des éléments optiques déviant le faisceau (121A), les éléments optiques déviant le faisceau (121A) étant disposés de telle sorte que des faisceaux de lumière soient déviés vers le convertisseur d'image commun (120) pour produire l'image en section transversale (30A).

7. Dispositif (1) selon l'une quelconque des revendications 1 à 5, **caractérisé en ce que** les premiers et deuxièmes moyens de détection d'image présentent un convertisseur d'image commun (120), et **en ce que** les deuxièmes moyens de détection d'image comprennent des éléments optiques déviant le faisceau (121E), les éléments optiques déviant le faisceau (121E) étant disposés de telle sorte que des faisceaux de lumière soient déviés vers le convertisseur d'image commun (120) pour produire l'image en projection (3A).

8. Dispositif (1) selon l'une quelconque des revendications 1 à 7, **caractérisé en ce que** les premiers moyens de détection d'image sont prévus pour détecter et mémoriser une deuxième image en section transversale (30B) de la région partielle de la partie en section transversale (4) éclairée par le premier projecteur de lumière (11), à partir d'une deuxième position en dehors du faisceau de rayons (2), simultanément à la détection de la première image en section transversale (30A), la première position et la deuxième position se situant sur des côtés différents d'un plan situé dans le faisceau de rayons (2).

9. Dispositif (1) selon la revendication 8, **caractérisé en ce que** les premiers moyens de détection d'image comprennent un convertisseur d'image (120) et **en ce que** les premiers moyens de détection d'image comprennent des éléments optiques déviant le faisceau (121A, 121B), les premiers des éléments optiques déviant le faisceau (121A) étant disposés dans la première position de telle sorte que des faisceaux de lumière soient déviés vers le convertisseur d'image (120) pour produire la première image en section transversale (30A), et les deuxièmes des éléments optiques déviant le faisceau (121B) étant disposés dans la deuxième position de telle sorte que des faisceaux de lumière soient déviés vers le convertisseur d'image (120) pour produire la deuxième image en section transversale (30B).

10. Dispositif (1) selon l'une quelconque des revendications 1 à 9, **caractérisé en ce qu'**il comprend un ou plusieurs deuxièmes projecteurs de lumière supplémentaires (16) pour projeter des marques de lumière (36) sur l'oeil (3), et **en ce que** les deuxièmes moyens de détection d'image sont synchronisés avec le premier projecteur de lumière (11) et avec les deuxièmes projecteurs de lumière (16) de telle sorte que lors de la détection et de la mémorisation de l'image en projection (3A) de l'oeil (3), l'image (4A) de la partie en section transversale (4) éclairée par premier projecteur de lumière (11) et une image des marques de lumière (36) projetées par les deuxièmes projecteurs de lumière (16) soient détectées et mémorisées conjointement.

11. Dispositif (1) selon l'une quelconque des revendications 1 à 10, **caractérisé en ce qu'**il comprend un corps d'écran (17) avec un motif visible (17'), ledit corps d'écran (17) étant disposé de telle sorte que le motif visible (17'), lors de l'application du dispositif (1), soit situé sur un côté du corps d'écran (17) tourné vers l'oeil (3), le corps d'écran (17) étant disposé de telle sorte que le faisceau de rayons (2) puisse être projeté sans obstruction à travers la partie en section transversale (4) de l'oeil (3) et que l'image en section transversale (30A) et l'image en projection (3A) puissent être détectées sans obstruction par les premiers, respectivement les deuxièmes, moyens de détection d'image.

12. Dispositif (1) selon l'une quelconque des revendications 1 à 11, **caractérisé en ce qu'**il comprend des moyens d'entraînement (15) pour faire tourner le premier projecteur de lumière (11) et les premiers et deuxièmes moyens de détection d'image essentiellement autour d'une normale à la surface de l'oeil (3) tournée vers le premier projecteur de lumière (11) ou pour les déplacer essentiellement perpendiculairement à cette normale.

13. Dispositif (1) selon l'une quelconque des revendications 1 à 12, **caractérisé en ce que** le premier projecteur de lumière (11) est configuré de telle sorte qu'il projette le faisceau de rayons (2) sous forme de fente de lumière.

14. Procédé de mesure ophtalmologique, comprenant :
- la projection d'un faisceau de rayons (2) à travers une partie en section transversale (4) d' un oeil (3), notamment à travers une partie en section transversale (4) de la cornée de l'oeil (3), au moyen d'un premier projecteur de lumière (11),
- la détection d'une image en section transversale (30A) d'au moins une région partielle de la partie en section transversale (4) éclairée par le premier projecteur de lumière (11), à partir d'une première position en dehors du faisceau de rayons (2), au moyen de premiers moyens de détection d'image qui sont disposés suivant un système Scheimpflug par rapport au faisceau de rayons (2),
- la détection d'une image en projection (3A) de l'oeil (3), au moyen de deuxièmes moyens de détection d'image de telle sorte que l'image en projection (3A) comprenne une image (4A) de la partie en section transversale (4) éclairée par le premier projecteur de lumière (11),
**caractérisé par**
- la mémorisation de l'image en section transversale (30A) détectée et de l'image en projection (3A) détectée de manière associée mutuellement dans une mémoire de données, et
- la détermination de la position de l'image en section transversale (30A) mémorisée par rapport à l'oeil (3) sur la base de l'image en projection (3A) mémorisée de manière associée.

15. Procédé selon la revendication 14, **caractérisé en ce que** l'image en section transversale (30A) mémorisée est positionnée sur la base de l'image en projection (3A) mémorisée de manière associée, par rapport à des images en section transversale de l'oeil (3) préalablement mémorisées.

16. Procédé selon l'une quelconque des revendications 14 ou 15, **caractérisé en ce que** l'épaisseur (d) de la partie en section transversale (4) de l'oeil (3) éclairée par le premier projecteur de lumière (11) est déterminée sur la base de l'image en projection (3A) mémorisée.

17. Procédé selon l'une quelconque des revendications 14 à 16, **caractérisé en ce que** les deuxièmes moyens de détection d'image et le premier projecteur de lumière (11) sont disposés de telle sorte que l'axe optique des deuxièmes moyens de détection d'image coïncide avec le faisceau de rayons (2) s'étendant à travers la partie en section transversale (4).

18. Procédé selon l'une quelconque des revendications 14 à 17, **caractérisé en ce que** les premiers et deuxièmes moyens de détection d'image sont disposés à chaque fois de telle sorte que leurs axes optiques se situent dans un plan commun.

19. Procédé selon l'une quelconque des revendications 14 à 18, **caractérisé en ce que** les premiers moyens de détection d'image sont pourvus d'éléments optiques déviant le faisceau (121A), les éléments optiques déviant le faisceau (121A) étant disposés de telle sorte que des faisceaux de lumière soient déviés vers un convertisseur d'image (120) utilisé en commun avec les deuxièmes moyens de détection d'image pour produire l'image en section transversale (30A).

20. Procédé selon l'une quelconque des revendications 14 à 18, **caractérisé en ce que** les deuxièmes moyens de détection d'image sont pourvus d'éléments optiques déviant le faisceau (121E), les éléments optiques déviant le faisceau (121E) étant disposés de telle sorte que des faisceaux de lumière soient déviés vers un convertisseur d'image (120) utilisé en commun avec les premiers moyens de détection d'image pour produire l'image en projection (3A).

21. Procédé selon l'une quelconque des revendications 14 à 20, **caractérisé en ce que**, en même temps que la détection de la première image en section transversale (30A), une deuxième image en section transversale (30B) de la région partielle de la partie en section transversale (4) éclairée par le premier projecteur de lumière (11) est détectée et mémorisée au moyen des premiers moyens de détection d'image à partir d'une deuxième position en dehors du faisceau de rayons (2), la première position et la deuxième position étant fixées sur des côtés différents d'un plan situé dans le faisceau de rayons (2).

22. Procédé selon la revendication 21, **caractérisé en ce que** des premiers éléments optiques déviant le faisceau (121A) des premiers moyens de détection d'image sont disposés dans la première position de telle sorte qu'ils dévient des faisceaux de lumière vers un convertisseur d'image (120) des premiers moyens de détection d'image pour produire la première image en section transversale (30A), et **en ce que** des deuxièmes éléments optiques déviant le faisceau (121B) des premiers moyens de détection d'image sont disposés dans la deuxième position de telle sorte qu'ils dévient des faisceaux de lumière vers le convertisseur d'image (120) des premiers moyens de détection d'image pour produire la deuxième image en section transversale (30B).

23. Procédé selon l'une quelconque des revendications 14 à 22, **caractérisé en ce que** des marques de lumière (36) sont projetées sur l'oeil (3) au moyen d'un ou de plusieurs deuxièmes projecteurs de lumière supplémentaires (16), et **en ce que** les deuxièmes moyens de détection d'image sont synchronisés avec le premier projecteur de lumière (11) et avec les deuxièmes projecteurs de lumière (16) de telle sorte que lors de la détection et de la mémorisation de l'image en projection (3A) de l'oeil (3), l'image (4A) de la partie en section transversale (4) éclairée par le premier projecteur de lumière (11) et une image des marques de lumière (36) projetées par les deuxièmes projecteurs de lumière (16) soient détectées et mémorisées conjointement.

24. Procédé selon l'une quelconque des revendications 14 à 23, **caractérisé en ce qu'**un corps d'écran (17) avec un motif visible (17') est disposé de telle sorte que le motif visible (17') soit tourné vers l'oeil (3) et que le faisceau de rayons (2) puisse être projeté sans obstruction à travers la partie en section transversale (4) de l'oeil (3) et que l'image en section transversale (30A) et l'image en projection (3A) puissent être détectées sans obstruction par les premiers, respectivement les deuxièmes, moyens de détection d'image.

25. Procédé selon l'une quelconque des revendications 14 à 24, **caractérisé en ce que** le premier projecteur de lumière (11) et les premiers et deuxièmes moyens de détection d'image sont tournés essentiellement autour d'une normale à la surface de l'oeil (3) tournée vers le premier projecteur de lumière (11) ou sont déplacés essentiellement perpendiculairement à cette normale.

26. Procédé selon l'une quelconque des revendications 14 à 25, **caractérisé en ce que** le premier projecteur de lumière (11) projette le faisceau de rayons (2) sous forme de fente de lumière.

27. Procédé selon l'une quelconque des revendications 14 à 26, **caractérisé en ce que** les deuxièmes moyens de détection d'image sont disposés de telle sorte que leur axe optique coïncide avec l'axe optique (Z) de l'oeil (3) ou s'étende essentiellement parallèlement à l'axe optique (Z) de l'oeil (3).

28. Procédé selon l'une quelconque des revendications 14 à 26, **caractérisé en ce que** les deuxièmes moyens de détection d'image sont disposés de telle sorte que leur axe optique coïncide avec l'axe de vision de l'oeil (3) ou s'étende essentiellement parallèlement à l'axe de vision de l'oeil (3).
